Europäisches Patentamt

European Patent Office

Office européen des brevets

(11) Publication number: **0 387 840 B1**

## EUROPEAN PATENT SPECIFICATION

(45) Date of publication of patent specification: **13.07.94** (51) Int. Cl.⁵: **C12M 1/36**, C12N 5/00

(21) Application number: **90104822.3**

(22) Date of filing: **14.03.90**

---

(54) **Method and apparatus for controlling cultivation conditions for animal cells.**

---

(30) Priority: **14.03.89 JP 59721/89**
**14.07.89 JP 180196/89**

(43) Date of publication of application:
**19.09.90 Bulletin 90/38**

(45) Publication of the grant of the patent:
**13.07.94 Bulletin 94/28**

(84) Designated Contracting States:
**DE GB**

(56) References cited:
**WO-A-88/01643**
**DE-A- 2 657 209**
**FR-A- 2 578 266**
**GB-A- 1 090 758**

**BIOTECHNOLOGY & BIOENGINEERING,vol. 27, no. 6, June 1985, New York, NY (US); T. TSUCHIDA et al., pp. 837-841&NUM;**

**PATENT ABSTRACTS OF JAPAN, vol. 13, no. 29 (C-562)[3377], 23 January 1989&NUM;**

(73) Proprietor: **HITACHI, LTD.**
**6, Kanda Surugadai 4-chome**
**Chiyoda-ku, Tokyo 100(JP)**

(72) Inventor: **Takeuchi, Kouji**
**5-329, 3-290-1 Matsugaoka**
**Nagareyama-shi, Chiba(JP)**
Inventor: **Kawaguchi, Hideo**
**C-204, 2-32, Koyasucho**
**Hachioji-shi, Tokyo(JP)**
Inventor: **Ishibashi, Tadashi**
**5-17-1-2, Jousuihoncho**
**Kodaira-shi, Tokyo(JP)**
Inventor: **Shimizu, Norio**
**4-6-2 Honcho**
**Kokubunji-shi, Tokyo(JP)**

(74) Representative: **Patentanwälte Beetz - Timpe - Siegfried Schmitt-Fumian - Mayr**
**Steinsdorfstrasse 10**
**D-80538 München (DE)**

---

EP 0 387 840 B1

# Description

This invention relates to a method and an apparatus for controlling cultivation conditions for animal cells.

Animal cells can produce proteins which cannot be produced by recombinant microorganisms, for example, those to which oligosaccharides are bound, those having high molecular weights and those having complicated stereochemical structures. Therefore, it has attracted scientific attention to produce these proteins by culturing animal cells. In particular, there has been an urgent demand to provide a process for the mass production of animal cells.

Technical problems observed in the high-density cultivation of cells involve the removal of proliferation inhibiting factors such as lactic acid and ammonia which are metabolites secreted by the cells. It is known that lactic acid and ammonia inhibit the proliferation of cells (see T. Kimura et al., Journal of Fermentation Technology, 65 (3), 341-344 (1987)). Thus there has been proposed a process for perfusion culture wherein fresh medium is continuously supplied while used medium is withdrawn so as to remove these proliferation inhibiting factors (see JP-A-36915/1986 and JP-A-257181/1986). In this perfusion cultivation process, the rates of feeding the fresh medium and withdrawing the used one are controlled mainly based on the proliferation rate of the cells. Thus it has been a practice that when this rate decreases, the rates of feeding the fresh medium and withdrawing the used one are increased so as to dilute the proliferation inhibiting factors (see M. Tokashiki et al., Kagaku Kogaku Ronbunshu, 14 (3), 337-341 (1988)).

The above mentioned prior art, wherein the proliferation inhibiting factors are diluted by feeding the medium, is disadvantageous in that the economic efficiency of the substrate-containing medium is not considered and thus the consumption of a large amount of substrate-containing medium elevates the running cost.

The present inventors have found out that the lactic acid concentration in a culture medium of animal cells is indicative for the proliferation status of the cells, and the lactic acid concentration can be lowered by nutritionally starving the animal cells and thus allowing the animal cells to assimilate the lactic acid accumulated in the culture medium.

The term animal cells includes mammalian and particularly human cells.

Conventional microorganism culture systems can hardly be applied to the mass production of animal cells for the following reason. An animal cell has a cell membrane having a poor resistance against shearing forces caused by stirring. Serum contained in a culture medium would lead to foaming during aeration. The long generation time of animal cells requires maintaining the system axenic for several tens of hours. It is particularly required to maintain a sensor for measuring the dissolved oxygen concentration in the culture medium stable for a long time, since the cultivation should be continued for a long time. However, an oxygen sensor would be damaged by heat or a change in pressure during steam sterilization. Further, the prolonged use of an oxygen sensor would sometimes cause drying up of liquid in the sensor or formation of a protein membrane at the sensor/liquid interface, which make the measurement impossible. When these troubles make the measurement of the dissolved oxygen concentration impossible, the controlled cultivation cannot be continued any more. Excessive supply of oxygen would be toxic for the animal cells, while shortage of oxygen would lower the proliferation.

In addition, there are no known optimum cultivation conditions for animal cells.

The failure in the measurement of the dissolved oxygen concentration and the lack of any procedure for determining the growth activity of animal cells makes it difficult to continue the cultivation for a prolonged period of time.

WO 88/01643 describes a method of culturing cells wherein, to provide a maximum growth of the cell line in a continuous culture system, the pH level is maintained corresponding to a pH value occuring at the time point of greatest increase in the cell number in a static culture, and the glucose arid lactate levels are also maintained in the continuous culture system corresponding to the glucose and lactate levels at a time point of greatest increase in the cell number under static culture conditions.

It has further been known from Biotechnology and Bioengineering 27, No. 6 (1985) 837-841, to monitor the lactate production and the glucose consumption during culturing, wherein the lactate production is detected by means of a lactate electrode comprising an immobilized lactate oxidase membrane.

In addition thereto, it has been known from JP-A-63-233780 to apply a feedback control of a high-density culture of animal cells by controlling the addition of acetic acid.

The determination and use of the ratio of lactate production to substrate consumption for feedback controlling animal cell cultures has not been indicated in the prior art publications.

Accordingly, it is the object of the present invention to provide a method and an apparatus for controlling cultivation conditions for animal cells which allow the high-density cultivation of animal cells and whereby the concentration of proliferation inhibiting factors can be efficiently decreased while efficiently utilizing the substrate-containing medium so that animal cells can be stably cultured for a long time.

The above-mentioned object is achieved according to the claims. The dependent claims relate to preferred embodiments.

The method according to the present invention for controlling the cultivation conditions for animal cells in a culture medium containing a substrate is characterized by

- measuring the lactic acid concentration $c_l$ and the substrate concentration $c_s$ of the culture medium at predetermined times defining predetermined time periods $\Delta t$, or continuously,
- calculating the rate L of lactic acid formation from the concentration change $\Delta c_l$ during the time periods $\Delta t$ between subsequent predetermined times as $\Delta c_l / \Delta t$, or continuously as $dc_l/dt$,
- calculating the rate S of substrate consumption from the concentration change $\Delta c_s$ during the same predetermined time periods $\Delta t$ as $\Delta c_s / \Delta t$, or continuously as $dc_s/dt$,
- calculating the respective ratio L/S according to the relations $L/S \approx \Delta c_l / \Delta c_s$ or $L/S = dc_l/dc_s$, and
- controlling the dissolved oxygen concentration and/or the substrate concentration $c_s$ in the culture medium such as to keep the ratio L/S at or below a predetermined upper limit value A depending on the kind of the animal cells.

According to a preferred embodiment, the controlling of the dissolved oxygen concentration and/or the substrate concentration $c_s$ in the culture medium is performed when the ratio L/S is or tends to be higher than the predetermined upper value A.

In the case of discontinuous measurement or detection of the lactic acid concentration $c_l$ and the substrate concentration $c_s$ at predetermined times defining predetermined time periods $\Delta t$, L and S are defined as follows:

$$L \approx \left| \frac{\Delta c_l}{\Delta t} \right|, \text{ and}$$

$$S \approx \left| \frac{\Delta c_s}{\Delta t} \right|,$$

L representing the rate of lactic acid formation, and S representing the rate of substrate consumption, based on the same times or time periods $\Delta t$.

In accordance therewith, the ratio L/S is defined as follows:

$$L/S \approx \frac{\left| \frac{\Delta c_l}{\Delta t} \right|}{\left| \frac{\Delta c_s}{\Delta t} \right|} \approx \left| \frac{\Delta c_l}{\Delta c_s} \right|.$$

In the case of continuous measurement of the lactic acid concentration $c_l$ and the substrate concentration $c_s$, the differential quotients

$$L = \frac{dc_l}{dt} \quad \text{and} \quad S = \frac{dc_s}{dt}$$

are determined, and the ratio L/S is calculated according to

$$L/S = \frac{\left| \frac{dc_l}{dt} \right|}{\left| \frac{dc_s}{dt} \right|} = \left| \frac{dc_l}{dc_s} \right|.$$

In case glucose is used as the substrate, the glucose concentration $c_g$ is determined in the same manner as defined above for the substrate concentration $c_s$, and accordingly, the rate G of glucose consumption is

$$G \approx \left| \frac{\Delta c_g}{\Delta t} \right| = \left| \frac{dc_g}{dt} \right|,$$

and the ratio L/S corresponds in this case to

$$L/G \approx \left| \frac{\Delta c_l}{\Delta c_g} \right| = \left| \frac{dc_l}{dc_g} \right|.$$

In the case of a constant lactic acid formation rate and a constant substrate (glucose) consumption rate,

$$\left|\frac{\Delta c_1}{\Delta t}\right| = \left|\frac{dc_1}{dt}\right| , \text{ and}$$

$$\left|\frac{\Delta c_s}{\Delta t}\right| = \left|\frac{dc_s}{\Delta t}\right| \quad \left(\left|\frac{\Delta c_g}{\Delta t}\right| = \left|\frac{dc_g}{dt}\right|\right),$$

and accordingly,
L/S is in this case

$$L/S = \left|\frac{\Delta c_1}{\Delta c_s}\right|,$$

and
L/G is

$$L/G = \left|\frac{\Delta c_1}{\Delta c_g}\right|.$$

In accordance with a preferred embodiment, the dissolved oxygen concentration and/or the substrate concentration $c_s$ ($c_g$) are controlled by stepwise supplying oxygen and/or substrate (glucose) to the culture medium at predetermined times, and the ratio L/S (L/G) is determined for each time period $\Delta t$ between these predetermined times.

The ratio L/S (L/G) must be kept within a predetermined, fixed range defined as to be

O < L/S (L/G) ≤ A,

A being the upper limit value particularly depending on the kind of the cultivated animal cells, and preferably being 0.6.

By means of these measures, the lactic acid concentration in the culture medium can be controlled and kept at or below the predetermined value A.

That controlling is preferably performed by increasing the dissolved oxygen concentration and/or by decreasing the substrate concentration $c_s$ - (glucose concentration $c_g$) in the culture medium.

It may be preferred to supply oxygen to the culture medium continuously for the entire culturing time.

In accordance with a preferred embodiment of the present method, the animal cells are periodically separated from the culture medium and put into a new culture medium, preferably into a new culture medium having a higher substrate/glucose concentration than the used culture medium; this is repeatedly carried out during the entire culturing time.

The lactic acid concentration in the culture medium can be lowered e.g. by temporarily starving the animal cells nutritionally and thus allowing the animal cells to assimilate lactic acid secreted by the animal cells to thereby control the lactic acid concentration in the culture medium to be below a predetermined value, preferably below 2.5 g/l.

In the above mentioned cultivation process, cells capable of expressing a gene of an enzyme which assimilates lactic acid may be used as the animal cells. It is preferable to use cardiac type lactate dehydrogenase as the enzyme which assimilates lactic acid.

Further, cells originating from liver, and more particularly, cells of the rat ascites hepatoma cell JTC-1 strain may be used. This strain which is described in Journal of Experimental Medicine, 28 (2), 115-117 (1958) and has been authorized by the Japanese Society of Tissue Culture, can be readily obtained.

In the cultivation process of the present invention, the predetermined value of the lactic acid concentration may vary depending on, for example, the animal cells to be used, the D cell concentration in the cultivation, and the kind of the medium.

Reducing the substrate/glucose concentration leads to a reduction of the cell growth and induces lactic acid assimilation when the lactic acid concentration $c_l$ of the culture medium reaches a predetermined value. This is performed preferably when the lactic acid concentration $c_l$ reaches 2.5 g/l. In this connection, it is preferable to add, throughout the entire cultivation, substrate/glucose until the lactic acid concentration $c_l$ reaches the predetermined value.

The reduction of the substrate/glucose concentration is advantageously performed by controlling or by stopping the addition of substrate/glucose.

Further, the invention provides an apparatus for controlling cultivation conditions for animal cells in a culture medium containing a substrate which is particularly suited for carrying out the above-described method; the apparatus comprises

- a fermentor for the culture medium, preferably provided with a stirrer,
- a substrate tank connected to the fermentor by means of a conduit comprising a control valve,
- a cell/culture medium separator provided at the discharge side of the fermentor,
- a culture medium supernatant pump provided in the discharge conduit of the fermentor,

- lactic acid concentration sensing means provided at or in the fermentor,
- substrate concentration sensing means provided at or in the fermentor, and
- an operation controller for controlling the control valve and receiving the output signals of the lactic acid concentration sensing means and the substrate concentration sensing means, and being adapted
  - to determine the rate L of lactic acid formation,
  - to determine the rate S of substrate consumption,
  - to calculate the ratio L/S, and
  - to control the substrate concentration $c_s$ in the culture medium via the supply of substrate by means of the control valve such as to keep the ratio L/S within at or below a predetermined upper limit value A.

In accordance with another preferred embodiment, this apparatus further comprises
- means for supplying oxygen to the fermentor, and
- glucose concentration sensing means provided at or in the fermentor and connected to the operation controller.

The oxygen supply means advantageously comprise an oxygen supply conduit and a control valve provided therein and being controlled by the operation controller.

The operation controller is adapted to carry out the above-described variants of the method of this invention by controlling the supply of substrate and/or of oxygen by means of the substrate control valve and/or the oxygen control valve, and eventually the discharge of the culture medium and the separation of the culture medium supernatant by means of the cell/culture medium separator, on the basis of the output signals of the lactic acid concentration sensing means and optionally also the glucose concentration sensing means.

The operation controller calculates the ratio L/S (L/G) based on the sensor input data and compares the ratio thus calculated with the fixed range which has been preliminarily input, and the dissolved oxygen concentration and/or the glucose concentration in the culture medium is controlled based on the result of this comparison.

The features and advantages of the present invention will become more clear from the following detailed description of preferred embodiments shown in the drawings, wherein:

Figs. 1(a) and 1(b), 2(a) and 2(b), 3(a) and 3(b), 4(a) and 4(b), and 5(a) and 5(b) show experimental results of animal cell cultivation examples;

Fig. 6 is a schematic view of an apparatus of the present invention;

Figs. 7(a) and 7(b) show results of an example of the present invention;

Figs. 8(a) - 8(c) are graphs showing that the JTC-1 strain will assimilate lactic acid;

Fig. 9 is another example of an apparatus of the present invention; and

Figs. 10(a) - 10(c) are graphs showing the results of using JTC-1 strain with the apparatus of Fig. 9, using lactic acid as an indicator.

Under aerobic conditions, animal cells acquire energy by converting glucose into carbon dioxide and water via the tricarboxylic acid cycle. The use of the tricarboxylic acid cycle is suitable for the growth of animal cells, and 38 mol of adenosine triphosphate (ATP) are formed from 1 mol of glucose in this cycle. Under conditions where oxygen is insufficient, on the other hand, animal cells acquire energy by converting glucose into lactic acid via the glycolysis pathway depending on the Pasteur effect. In this latter case, only 2 mol of ATP can be formed from 1 mol of glucose. Thus the exclusive use of the glycolysis pathway results in an extremely low efficiency of the utilization of glucose. The conditions of the supply of oxygen and the growth activity of the cells are determined by using the ratio of the formed lactic acid to the consumed glucose (L/G) as an indication.

Even though a sufficient amount of oxygen is supplied, furthermore, lactic acid would be formed by Crabtree's effect when a culture medium contains glucose at a high concentration (cf. Crabtree, Biochemistry 23, 537-545 (1929)). In this case, the cells acquire energy by using the glycolysis pathway exclusively. Thus the growth activity of the cells is low.

The growth activity of cells is determined by measuring the glucose concentration and lactic acid concentration in the culture medium and using the ratio of formed lactic acid to consumed glucose (L/G) obtained from these data as an indication to thereby get an information about the cultivation conditions and control the same.

The preferred range of the L/G ratio has been established through the following cultivation experiments conducted under various conditions, and is found to be $0 < L/G \leq 0.6$.

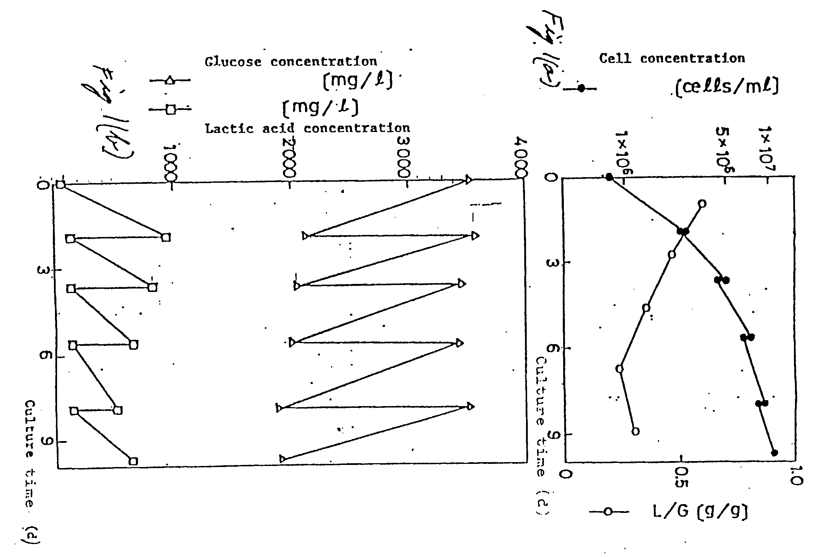

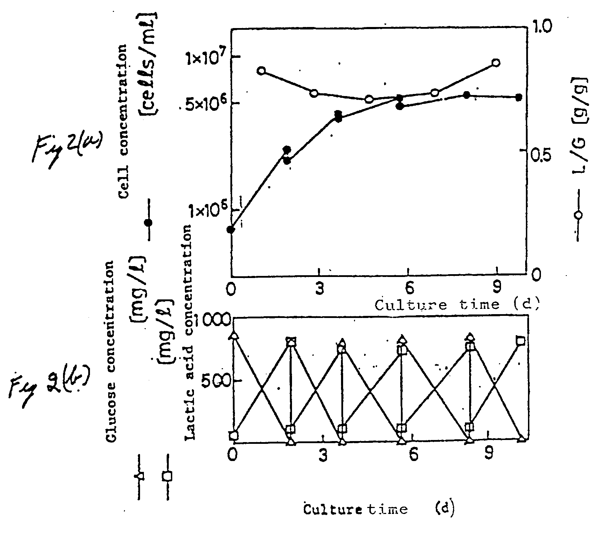

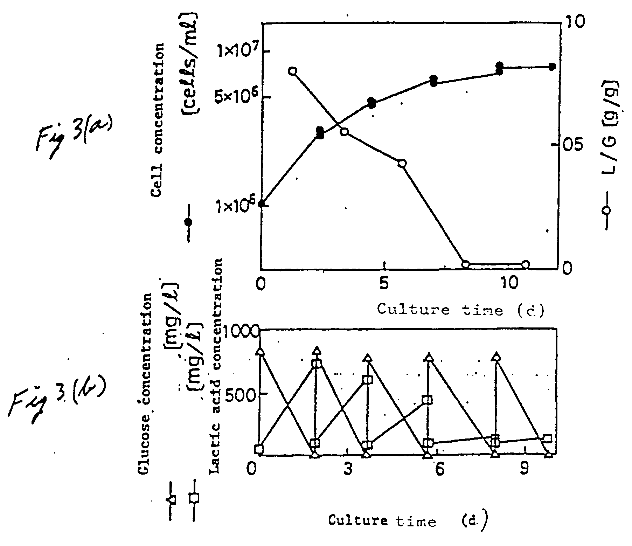

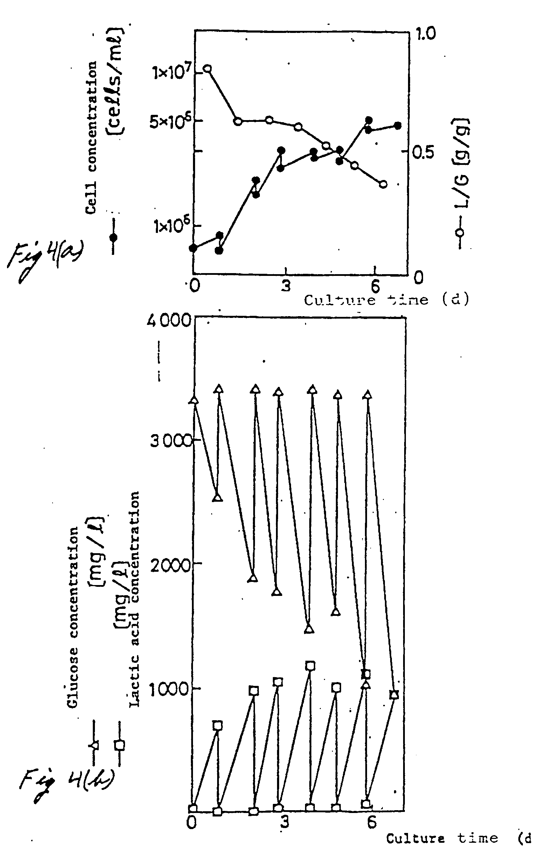

An air filter (7.5 cm$^2$) was attached to the lid of a 200-ml tapping culture flask in such a manner that an oxygen-containing gas stream was introduced therethrough into the container. 80 ml of a DM160-AU medium (mfd. by Kyokuto Seiyaku Kogyo K.K.) containing 10 % of new born bovine serum was fed into the container and then inoculated with seed cells of JTC-1 strain (rat abdominal hepatoma cells) at a concentration of $1 \times 10^6$ cells/ml. Then the cells were cultured with stirring

at 400 min$^{-1}$ at 37 °C and with use of a $CO_2$ incubator. The culture medium was centrifuged at 1200 min$^{-1}$ for 10 min every two days, and the centrifugally separated cells were placed in a new medium, which corresponds to an exchange of the whole medium. The initial glucose concentration of the culture medium was adjusted to 3500 mg/l at the time of each exchange. Before and after each exchange of the whole medium, 2-ml portions of the culture medium were sampled, and the glucose concentration and lactic acid concentration of each sample were measured with an enzyme sensor. Further the cell concentration was measured with a hemacytometer. Fig. 1 shows the results obtained. The cells grew well, and the cell concentration reached 1 x 10$^7$ cells/ml on the tenth day. The ratio L/G was as high as 0.5 at the early stage of the cultivation, and then slowly decreased and finally reached 0.3. Thus it was found that a ratio L/G of 0.6 or below indicated that the cultivation conditions were aerobic, and a sufficient amount of glucose was supplied, i.e., the cells showed a high growth activity, and the optimum cultivation conditions were established.

In the following examples, the conditions applied are the same except where differences are noted.

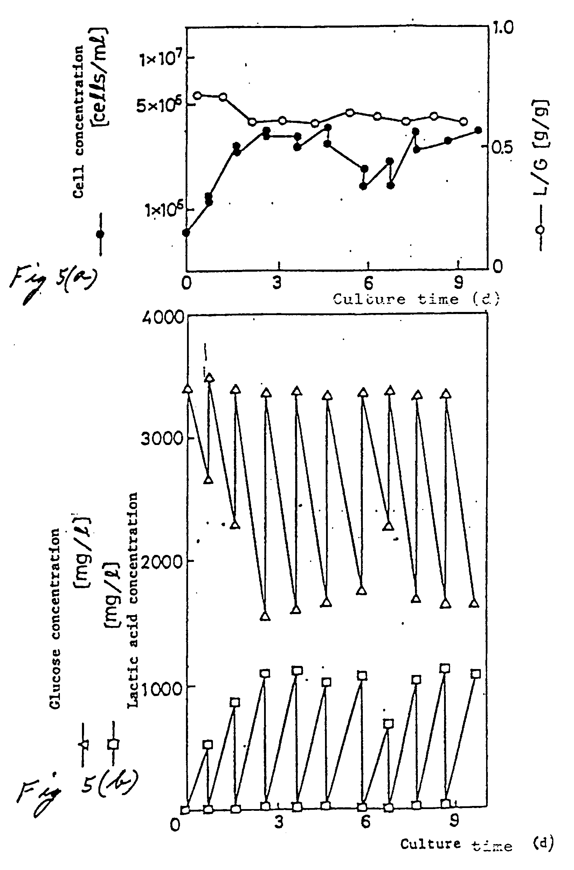

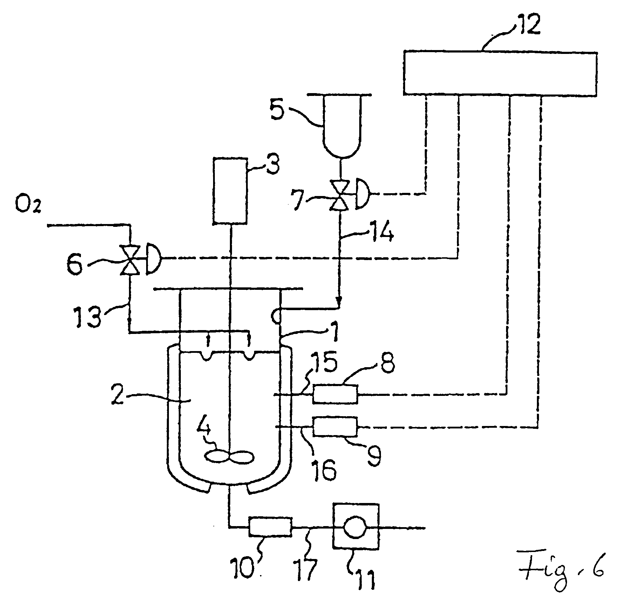

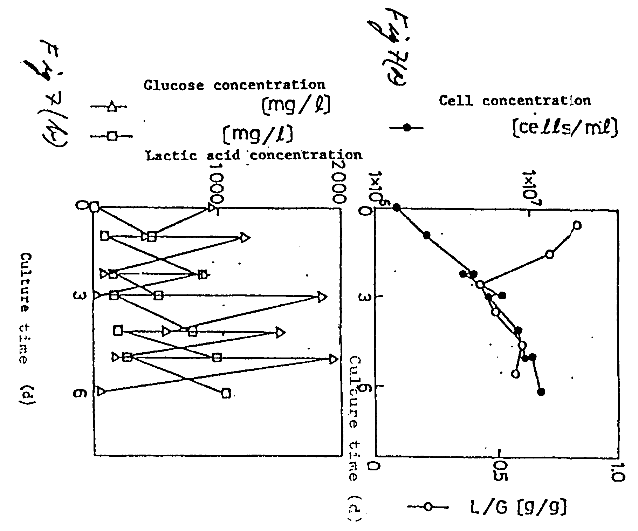

Next, the glucose concentration in the culture medium at the time of each exchange of the whole medium was adjusted to 1000 mg/l, and the air filter was removed. Namely, the cultivation was carried out under hermetically sealed conditions. Fig. 2 shows the results obtained. The final cell concentration was 5.5 x 10$^6$ cells/ml while the ratio L/G exceeded 0.6 throughout most of the cultivation period. Thus it was found that the growth activity of the cells was lowered when the ratio L/G exceeded 0.6 and the glucose concentration was not excessively high.

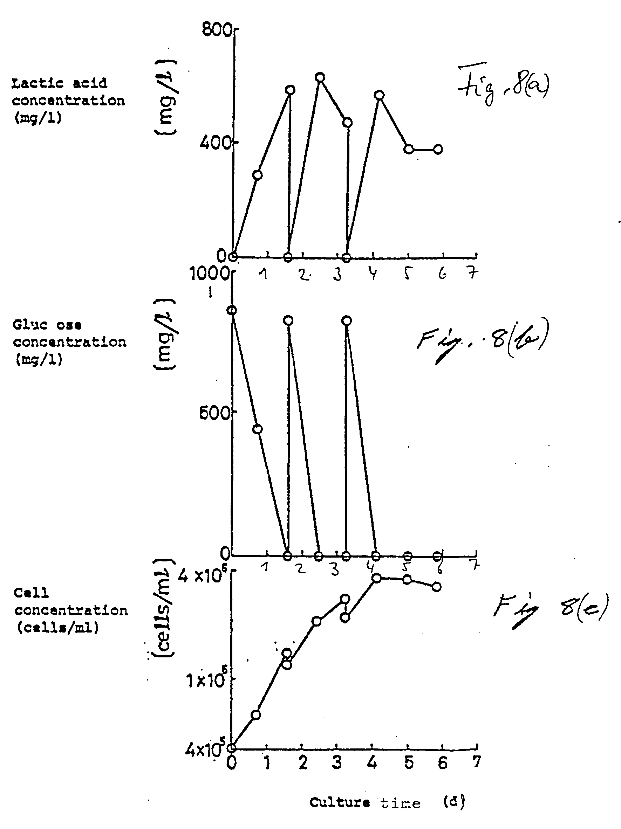

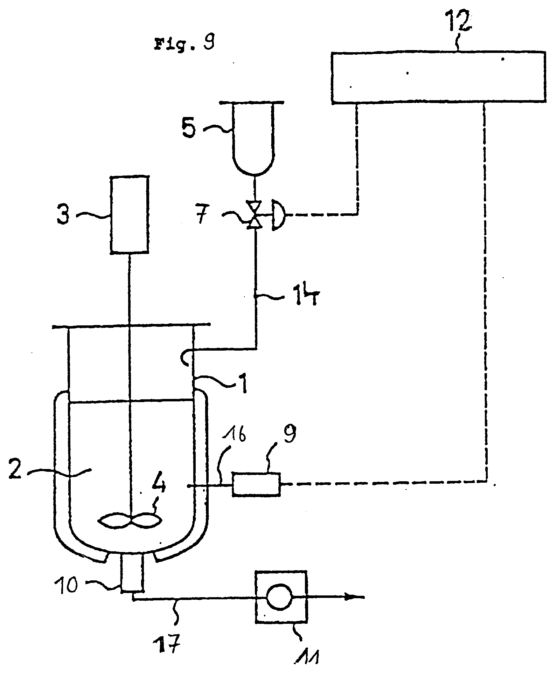

Next, the glucose concentration in the culture medium at the time of each exchange of the whole medium was adjusted to 1000 mg/l, and the air filter was attached to the lid. Fig. 3 shows the results. The final cell concentration was 7.5 x 10$^6$ cells/ml while the ratio L/G decreased with the lapse of time and reached zero on the eighth day simultaneously with the consumption of the glucose. Thus it was found that the glucose concentration of 1000 mg/l was insufficient for achieving a high-density cell concentration of 1 x 10$^7$ cells/ml or higher.

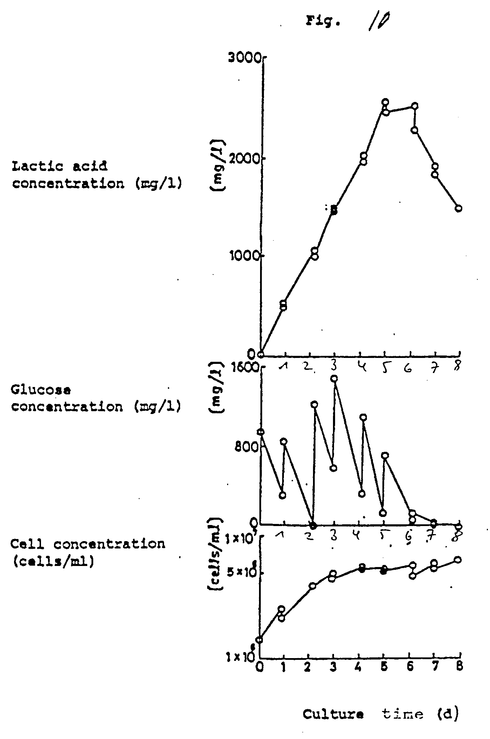

Then mouse hybridoma strain 11D-11-1 (parent strain: P3-U1) was cultured. An air filter was attached to the same tapping culture flask as the one described above to thereby introduce an oxygen-containing gas stream with variable oxygen concentrations into the container. 80 ml of a serum-free medium E-RDF + RD-1 (mfd. by Kyokuto Seiyaku Kogyo K.K.) was fed into the container and inoculated with seed cells of 11D-11-1 at a concentration of 1 x 10$^6$ cells/ml. Then the cells were cultured with stirring at 300 min$^{-1}$ at 37 °C with use of a $CO_2$ incubator. The culture medium was then centrifuged at 1200 min$^{-1}$ for 10 min every day to thereby exchange the whole medium. The initial glucose concentration of the culture medium was adjusted to 3423 mg/l at the time of each exchange. Before and after each exchange of the whole medium, 2-ml portions of the culture medium were sampled, and the glucose concentration and lactic acid concentration of each sample were measured with an enzyme sensor. Further the cell concentration was measured with a hemacytometer. Fig. 4 shows the obtained results. On the fourth day of the cultivation, the cell concentration reached 3 x 10$^6$ cells/ml, and the growth ceased when the L/G ratio exceeded 0.6. When the oxygen concentration of the oxygen-containing gas was adjusted to 60 %, however, the cell concentration reached 5 x 10$^6$ cells/ml, and the L/G ratio decreased to 0.4 on the seventh day. Thus it was found that a L/G ratio of 0.6 or below indicated that the cultivation was conducted under aerobic conditions and the cells showed a high growth activity, namely, conditions suitable for the growth were established.

Next, the cultivation was conducted with an oxygen concentration of the oxygen containing gas which was constantly kept at 20 %. Fig. 5 shows the results. The cell concentration reached 3 x 10$^6$ cells/ml on the third day but the cells did not grow any more. The L/G ratio exceeded 0.6 throughout the cultivation period. Thus it was found that the supply of oxygen was insufficient and the growth activity of the cells was lowered when the L/G ratio exceeded 0.6 and the glucose concentration was not excessively high.

The results of these Experimental Examples show that the growth conditions of cells can be determined from the consumed glucose and the formed lactic acid, particularly their ratio. The cells particularly grew well when the L/G ratio ranged from greater than zero to 0.6, which suggests that the supply of glucose and oxygen under such conditions might be suitable for the growth of the cells.

In the method for controlling cultivation conditions according to the present invention, therefore, the dissolved oxygen concentration and/or glucose concentration in the culture medium should be controlled when the L/G is outside a fixed range, which preferably is from greater than 0 to 0.6.

The control may be conducted by increasing the dissolved oxygen concentration of the culture medium and/or decreasing the glucose concentration thereof. When the L/G ratio exceeds 0.6, for example, the supply of oxygen is first increased, and it is examined whether the L/G ratio is lowered to 0.6. When the L/G ratio is still above 0.6, the control can be achieved by decreasing the supply of glucose.

An example of a cultivation with controlling according to the present invention will be described by reference to Fig. 6. A fermentor 1 contains a culture medium 2 in which cells may be optionally immobilized, for example, on a microcarrier. The cells are suspended in the culture medium 2 by a stirrer 4 comprising rotating stirring blades with use of a motor 3. Oxygen may be supplied by either overlay aeration, bubbling or membrane aeration. The oxygen-containing gas is fed into the fermentor 1 through a conduit 13, and the $O_2$ flow rate is controlled with a control valve 6. Preserve culture medium containing glucose or an aqueous solution of glucose is provided in a substrate tank 5. The glucose-containing solution is fed to the fermentor 1 through a conduit 14, and the flow rate thereof is controlled with a control valve 7. The cultivation may be conducted by, for example, semibatch culture or continuous culture. Continuous culture is suitable for mass production. The culture supernatant is separated from the cells by a cell/culture medium separator 10 and discharged out of the fermentor 1 through a discharge conduit 17 with use of a culture supernatant pump 11. The concentrations of lactic acid and glucose in the culture medium 2 may be measured by, for example, using an enzyme sensor or liquid chromatography. In this case, they are measured with a glucose concentration sensor 8 and a lactic acid concentration sensor 9, respectively, which are connected to the fermentor 1 by means of conduits 15 and 16, respectively. The output data from the sensors 8, 9 are input to an operation controller 12 where calculations are performed and control signals are generated according to the ratio L/G. Then the control signals are output to the control valves 6 and 7 to control the supply of oxygen and substrate, respectively.

The present invention is based on the finding that the ratio of the concentration of lactic acid formed in the culture medium to that of the consumed glucose (L/G) correlates to the dissolved oxygen concentration in the culture medium and to the growth conditions of the cells. Further, it had been found that the cells grew well when the L/G ratio was within a fixed range, in particular, from greater than 0 to 0.6. Accordingly, the cultivation conditions for animal cells may be appropriately controlled by maintaining the L/G ratio within that range. In the present invention, the ratio L/G is maintained within the predetermined range by controlling the dissolved oxygen concentration and/or the glucose concentration.

The present invention is further illustrated by way of the following example.

An air filter (7.5 cm$^2$) was attached to the lid of a 200-ml tapping culture flask in such a manner that an oxygen-containing gas stream was introduced through the filter into the container. 80 ml of DM160-AU medium containing 10 % of new born bovine serum was fed into the container and then inoculated with seed cells of the JTC-1 strain at a concentration of $1 \times 10^6$ cells/ml. Then the cells were cultured with stirring at 400 min$^{-1}$ at 37 °C, and with use of a $CO_2$ incubator. The culture medium was centrifuged at 1200 min$^{-1}$ for 10 min every day to thereby exchange the whole medium.

Before and after each exchange of the whole medium, 2-ml portions of the culture medium were sampled, and the glucose concentration and the lactic acid concentration of each sample were measured with an enzyme sensor. Further the cell concentration was measured with a hemacytometer. The glucose concentration of the medium to be exchanged was determined according to an assumed glucose consumption rate of the JTC strain of $1 \times 10^{-7}$ mg/cell x d. When the L/G ratio exceeded 0.6, this value was decreased. When the L/G ratio fell down to 0.4 or below, on the other hand, this value was increased for safety. Fig. 7 shows the results of the cultivation. The cell concentration reached $1 \times 10^7$ cells/ml on the fifth day. The L/G ratio was high on the first day, slowly decreased thereafter and reached 0.4 on the third day. Then the supply of glucose was increased, which caused an increase in the L/G ratio. Except for the first day, the L/G ratio was within the range of from 0 to 0.6, and the cells grew well. Thus a high density cultivation of $1 \times 10^7$ cells/ml was effected.

The lactic acid concentration may be controlled to be below a predetermined value by controlling the medium, particularly the flow rate of the substrate depending on the lactic acid concentration in the culture medium in, for example, the following manner with respect to Figures 8 and 10.

After initiation of the cultivation, the substrate is continuously added while the lactic acid concentration is lower than the predetermined value. The rate of feeding the substrate may be increased. When the lactic acid concentration exceeds a predetermined value, the addition of the substrate is ceased or the rate of its addition is lowered. Thus the lactic acid is assimilated by the animal cells, which lowers the lactic acid concentration.

Although the predetermined value of the lactic acid concentration may vary somewhat depending on the kind and concentration of the animal cells and the kind of the medium, it is generally preferable to be below 2.5 g/l.

Figs. 8(a) - 8(c) show the results of the cultivation of JTC-1 strain. 80 ml of DM-160AU medium containing 10 % of new born bovine serum was fed into a 200-ml tapping culture flask and then inoculated with seed cells of JTC-1 strain at a concentration of $4 \times 10^5$ cells/ml. Then the cells were cultured while stirring at 400 min$^{-1}$ and 37 °C in an incubator. After 1.6 and 3.2 days, the culture medium was centrifuged at 12,000 min$^{-1}$ for 10 min to separate the cells from the used medium; then, new medium was added to the cells, thereby exchanging the whole medium. 2-ml portions of the culture medium were sampled everyday to measure the glucose concentration and lactic acid concentration of each sample with an enzyme sensor. Further the cell concentration was measured with a hemacytometer. As a result, the cell concentration on the fourth day was found to be $4 \times 10^6$ cells/ml, showing an excellent growth. The glucose concentration decreased to 0 mg/l after 1.6, 2.4, and 4.1 days, and an obvious decrease in the lactic acid concentration was observed each time.

These results indicate that the cells used above obviously assimilate lactic acid when glucose used as the substrate is running out. Therefore, the assimilation of lactic acid may be appropriately effected by measuring the lactic acid concentration in the culture medium and decreasing the amount of the substrate to be added or stopping the addition of the substrate, when the lactic acid concentration exceeds the predetermined value as a way of controlling the medium, particularly adjusting the glucose concentration. When the lactic acid concentration falls below the predetermined value again, the amount of the substrate to be added may be increased, or the addition of the substrate may be started again.

The term "substrate" as used herein means a carbon source required in the cultivation of the animal cells, and examples thereof include glucose, fructose and galactose. The substrate may be either a solid or a liquid. A liquid substrate may be mixed in water or in another medium.

Now an example of the cultivation apparatus of the present invention will be described by reference to Fig. 9 where the same reference numerals designate the same elements as in Fig. 6. The fermentor 1 contains a culture medium 2 in which cells, which may be optionally immobilized on, for example, a microcarrier, are suspended. The cells are suspended by means of a stirrer 4 comprising rotating stirring blades and a motor 3. Oxygen may be supplied by either overlay aeration, bubbling or membrane aeration. A medium containing glucose dissolved therein or an aqueous solution of glucose is stored in the substrate tank 5. A solution containing substrate is fed to the fermentor 1 via the conduit 14, and the feed rate is controlled with the control valve 7. The cultivation may be conducted by, for example, semi-batch culture or continuous culture, of which the continuous culture is suitable for mass production. The culture supernatant is separated from the cells with the cell/culture medium separator 10 through filtration or sedimentation and withdrawn out of the fermentor 1 through the conduit 17 with the use of the culture medium supernatant pump 11. The lactic acid concentration in the culture medium 2 may be measured by, for example, using liquid chromatography or with an enzyme sensor as lactic acid concentration sensor 9 connected to the fermentor 1 through the conduit 16. The data thus obtained are input into the operation controller 12 for comparison and judgment, and then control signals are output to the control valve 7 to control the feed rate of the substrate. When the lactic acid concentration reaches a predetermined value, the amount of the substrate-containing solution to be fed is decreased, or the feeding is ceased while the amount of the culture supernatant to be withdrawn with the culture medium supernatant pump 11 is decreased, or the withdrawal is ceased. Thus the lactic acid is assimilated.

The cultivation method of the present invention can be applied to the cultivation of cells capable of producing useful substances, for example, monoclonal antibodies or lymphokines so as to efficiently produce said useful substances.

Animal cells secrete lactic acid which inhibits the proliferation of the cells. This acid is produced since the cells contain lactate dehydrogenase which produces lactic acid from pyruvic acid.

In the present invention, the lactic acid concentration in a culture medium is measured, and the amount of a substrate to be fed is controlled depending on the lactic acid concentration thus measured, based on the finding that animal cells, which are temporarily starved nutritionally, would assimilate lactic acid. When the lactic acid concentration falls below a predetermined value thereby the proliferation of the cells continues without being inhibited by lactic acid, which enables prolonged cultivation at a high cell density. Furthermore, the substrate to be added may be efficiently utilized thereby. Therefore, the method of the present invention is highly advantageous in the mass cultivation of animal cells.

To further illustrate the present invention, the following examples will be given with respect to Fig. 10.

In these examples, lactic acid secreted by cells was measured with an enzyme sensor, and the amount of the substrate to be fed was controlled to keep the lactic acid concentration in the culture medium below 2.5 g/l. 80 ml of DM-160AU medium containing 10 % of newborn bovine serum was fed into a 200-ml tapping culture flask and then inoculated with seed cells of JTC-1 strain at a concentration of $4 \times 10^5$ cells/ml. Then the cells were cultured with stirring at 400 min$^{-1}$ at 37 °C in a $CO_2$ incubator. A 100 g/l aqueous solution of glucose was used as the substrate to be added. An appropriate amount of the glucose solution was fed everyday until the lactic acid concentration reached 2.5 g/l. When the lactic acid concentration reached 2.5 g/l, the amount of the substrate to be fed was decreased, or the feeding was ceased and the cultivation was continued. The lactic acid concentration and the glucose concentration were measured everyday before and after feeding the substrate. The cell concentration was measured with a hemacytometer. Fig. 10 shows the results. The lactic acid concentration reached 2.5 g/l after five days, and the proliferation stopped when the cell concentration reached $5.5 \times 10^6$ cells/ml. Then the amount of the glucose to be fed was decreased at the fifth day, and the feeding thereof was ceased at the sixth day. As a result, it was confirmed that the cell concentration increased with a decrease in the lactic acid concentration. These facts suggest that the cell concentration can be increased, and the substrate-containing medium can be efficiently utilized by controlling the lactic acid concentration in the culture medium.

According to the present invention, the lactic acid concentration in a culture medium can be controlled in the cultivation of animal cells, which makes it possible to efficiently culture the animal cells at a high density while using less of the substrate-containing medium than in the prior art, because of the efficient utilization of the substrate.

The present invention makes it possible to stably control cultivation conditions for animal cells for a prolonged period of time.

**Claims**

1. A method for controlling cultivation conditions for animal cells in a culture medium containing a substrate,
   **characterized** by
   - measuring the lactic acid concentration ($c_l$) and the substrate concentration ($c_s$) of the culture medium at predetermined times defining predetermined time periods ($\Delta t$), or continuously,
   - calculating the rate (L) of lactic acid formation from the concentration change ($\Delta c_l$) during the time periods ($\Delta t$) between subsequent predetermined times as $\Delta c_l/\Delta t$, or continuously as $dc_l/dt$,
   - calculating the rate (S) of substrate consumption from the concentration change

($\Delta c_s$) during the same predetermined time periods ($\Delta t$) as $\Delta c_s/\Delta t$, or continuously as $dc_s/dt$,
   - calculating the respective ratio L/S according to the relations $L/S \approx \Delta c_l/\Delta c_s$ or $L/S = dc_l/dc_s$,
   and
   - controlling the dissolved oxygen concentration and/or the substrate concentration ($c_s$) in the culture medium such as to keep the ratio L/S at or below a predetermined upper limit value (A) depending on the kind of the cultivated animal cells.

2. The method according to claim 1, characterized in that glucose is used as substrate.

3. The method according to claim 1 or 2, characterized in that the dissolved oxygen concentration and/or the substrate concentration ($c_s$) are controlled by stepwise supplying oxygen and/or substrate to the culture medium at predetermined times, and the ratio L/S is determined for each time period ($\Delta t$) between these predetermined times.

4. The method according to any one of claims 1 to 3, characterized in that controlling the dissolved oxygen concentration and/or the substrate concentration ($c_s$) in the culture medium is performed when the ratio L/S is or tends to be higher than the predetermined limit value (A).

5. The method according to any one of claims 1 to 4, characterized in that the upper limit value A is 0.6.

6. The method according to any one of claims 2 to 5, characterized in that controlling is performed by increasing the dissolved oxygen concentration and/or by decreasing the glucose concentration ($c_g$) in the culture medium.

7. The method according to any one of claims 1 to 6, characterized in that oxygen is supplied to the culture medium continuously for the entire culturing time.

8. The method according to any one of claims 1 to 7, characterized in that during the entire culturing time, the animal cells are periodically separated from the culture medium and put into a new culture medium, preferably into a new culture medium having a higher substrate/glucose concentration than the used culture medium.

9. The method according to any one of claims 1 to 9, characterized in that controlling is performed by reducing the substrate/glucose concentration to thereby reduce cell growth and induce lactic acid assimilation when the lactic acid concentration ($c_l$) of the culture medium reaches a predetermined value, preferably 2.5 g/l.

10. The method according to claim 9, characterized in that throughout the cultivation substrate/glucose is added until the lactic acid concentration ($c_l$) reaches the predetermined value.

11. The method according to any one of claims 1 to 10, characterized in that controlling is performed by reducing the substrate/glucose concentration by controlling or by stopping the addition of substrate/glucose.

12. The method according to any one of claims 1 to 11, characterized by the use of animal cells of a type expressing a gene of an enzyme which assimilates lactic acid, preferably a cardiac type lactate dehydrogenase.

13. The method according to any one of claims 1 to 12, characterized in that animal cells originating from liver are used.

14. An apparatus for controlling cultivation conditions for animal cells in a culture medium containing a substrate, particularly for carrying out the method according to any one of claims 1 to 13, comprising
    - a fermentor (1) for the culture medium (2) preferably provided with a stirrer (4),
    - a substrate tank (5) connected to the fermentor (1) by means of a conduit (14) comprising a control valve (7),
    - a cell/culture medium separator (10) provided at the discharge side of the fermentor (1),
    - a culture medium supernatant pump (11) provided in the discharge conduit (17) of the fermentor (1),
    - lactic acid concentration sensing means (9) provided at or in the fermentor (1),
    - substrate concentration sensing means (8) provided at or in the fermentor (1), and
    - an operation controller (12) for controlling the control valve (7) and receiving the output signals of the lactic acid concentration sensing means (9) and the substrate concentration sensing means (8), and being adapted

    - to determine the rate (L) of lactic acid formation,
    - to determine the rate (S) of substrate consumption,
    - to calculate the ratio L/S, and
    - to control the substrate concentration ($c_s$) in the culture medium via the supply of substrate by means of the control valve (7) such as to keep the ratio L/S within at or below a predetermined upper limit value (A).

15. The apparatus according to claim 14, further comprising
    - means (6, 13) for supplying oxygen to the fermentor (1), comprising an oxygen supply conduit (13) and a control valve (6) provided in the conduit (13) and being controlled by the operation controller (12).

16. The apparatus according to claim 14 or 15, characterized in that the substrate concentration sensing means (8) are glucose concentration sensing means.

17. The apparatus according to any one of claims 14 to 16, characterized in that the operation controller (12) is adapted to control the discharge of the culture medium and the separation of the culture medium supernatant by means of the cell/culture medium separator (10) on the basis of the output signals of the lactic acid concentration sensing means (9) and the substrate concentration sensing means (8).

**Patentansprüche**

1. Verfahren zur Steuerung der Kultivierungsbedingungen für tierische Zellen in einem ein Substrat enthaltenden Kulturmedium, **gekennzeichnet** durch
    - Messen der Milchsäurekonzentration ($c_l$) und der Substratkonzentration ($c_s$) des Kulturmediums zu gegebenen Zeiten, die gegebene Zeitspannen ($\Delta t$) definieren, oder
    - Berechnen der Geschwindigkeit (L) der Milchsäurebildung aus der Konzentrationsänderung ($\Delta c_l$) während der Zeitspannen ($\Delta t$) zwischen aufeinanderfolgenden gegebenen Zeiten als $\Delta c_l/\Delta t$ oder kontinuierlich als $dc_l/dt$,
    - Berechnen der Geschwindigkeit (S) des Substratverbrauchs aus der Konzentrationsänderung ($\Delta c_s$) während derselben

gegebenen Zeitspannen ($\Delta t$) als $\Delta c_s/\Delta t$ oder kontinuierlich als $dc_s/dt$,

- Berechnen des jeweiligen Verhältnisses L/S gemäß den Beziehungen L/S $\approx$ $\Delta c_l/\Delta c_s$ oder L/S = $dc_l/dc_s$, und
- Steuern der Konzentration an gelöstem Sauerstoff und/oder der Substratkonzentration ($c_s$) im Kulturmedium, um je nach Art der kultivierten tierischen Zellen das Verhältnis L/S auf oder unter einem gegebenen oberen Grenzwert (A) zu halten.

2. Verfahren nach Anspruch 1, dadurch gekennzeichnet, daß als Substrat Glucose verwendet wird.

3. Verfahren nach Anspruch 1 oder 2, dadurch gekennzeichnet, daß die Konzentration des gelösten Sauerstoffs und/oder die Substratkonzentration ($c_s$) kontrolliert werden durch schrittweises Liefern von Sauerstoff und/ oder Substrat zum Kulturmedium zu gegebenen Zeiten, und daß das Verhältnis L/S für jede Zeitspanne ($\Delta t$) zwischen diesen gegebenen Zeiten bestimmt wird.

4. Verfahren nach einem der Ansprüche 1 bis 3, dadurch gekennzeichnet, daß die Steuerung der Konzentration an gelöstem Sauerstoff und/oder der Substratkonzentration ($c_s$) im Kulturmedium erfolgt, wenn das Verhältnis L/S größer als der gegebene Grenzwert (A) ist oder dazu neigt.

5. Verfahren nach einem der Ansprüche 1 bis 4, dadurch gekennzeichnet, daß der obere Grenzwert (A) 0,6 ist.

6. Verfahren nach einem der Ansprüche 2 bis 5, dadurch gekennzeichnet, daß die Steuerung durch Erhöhen der Konzentration an gelöstem Sauerstoff und/oder durch Verringern der Glucosekonzentration ($c_g$) im Kulturmedium erfolgt.

7. Verfahren nach einem der Ansprüche 1 bis 6, dadurch gekennzeichnet, daß während der gesamten Kultivierungszeit Sauerstoff kontinuierlich zum Kulturmedium geliefert wird.

8. Verfahren nach einem der Ansprüche 1 bis 7, dadurch gekennzeichnet, daß während der gesamten Kultivierungszeit die tierischen Zellen vom Kulturmedium periodisch getrennt und in ein neues Kulturmedium gegeben werden, vorzugsweise in ein neues Kulturmedium mit einer höheren Substrat /Glucosekonzentration als das gebrauchte Kulturmedium.

9. Verfahren nach einem der Ansprüche 1 bis 9, dadurch gekennzeichnet, daß die Steuerung durch Verringern der Substrat /Glucosekonzentration erfolgt, um hierdurch das Zellenwachstum zu verringern und eine Milchsäureassimilation eingeleitet wird, wenn die Milchsäurekonzentration ($c_l$) des Kulturmediums einen gegebenen Wert, vorzugsweise 2,5 g/l, erreicht.

10. Verfahren nach Anspruch 9, dadurch gekennzeichnet, daß während der gesamten Kultivierung Substrat/Glucose zugeführt wird, bis die Milchsäurekonzentration ($c_l$) den gegebenen Wert erreicht.

11. Verfahren nach einem der Ansprüche 1 bis 10, dadurch gekennzeichnet, daß die Steuerung durch Verringern der Substrat/Glucosekonzentration durch Steuern oder Unterbrechen der Hinzufügung von Substrat/Glucose erfolgt.

12. Verfahren nach einem der Ansprüche 1 bis 11, gekennzeichnet durch die Verwendung von tierischen Zellen eines Typs, der ein Gen eines Milchsäure assimilierenden Enzyms ausdrückt, vorzugsweise einer Lactatdehydrase vom Cardiactyp.

13. Verfahren nach einem der Ansprüche 1 bis 12, dadurch gekennzeichnet, daß von Leber stammende tierische Zellen verwendet werden.

14. Vorrichtung für die Steuerung der Kultivierungsbedingungen für tierische Zellen in einem ein Substrat enthaltenden Kulturmedium, insbesondere zur Durchführung des Verfahrens nach einem der Ansprüche 1 bis 13, mit

- einer vorzugsweise mit einem Rührwerk (4) versehenen Fermentiereinrichtung (1) für das Kulturmedium (2),
- einem Substrattank (5), der durch eine ein Steuerventil (7) enthaltende Leitung (14) mit der Fermentiereinrichtung (1) verbunden ist.
- einem Zellen/Kulturmedium-Abscheider (10), der an der Auslaßseite der Fermentiereinrichtung (1) angeordnet ist,
- einer Pumpe (11) für das Überstehende des Kulturmediums, die in der Auslaßleitung (17) der Fermentiereinrichtung (1) angeordnet ist,
- einer Erfassungseinrichtung (9) für die Milchsäurekonzentration, die an oder in der Fermentiereinrichtung (1) angeordnet ist,
- einer Erfassungseinrichtung (8) für die Substratkonzentration, die an oder in der Fermentiereinrichtung (1) angeordnet ist, und
- einer Betriebssteuereinrichtung (12) für die Steuerung des Steuerventils (7) und für den Empfang der Ausgangssignale der Erfassungseinrichtung (9) für die Milchsäurekonzentration und der Erfassungseinrichtung (8) für die Substratkonzentration, wobei die Betriebssteuereinrichtung sich eignet zur
- Bestimmung der Geschwindigkeit (L) der Milchsäurebildung,
- Bestimmung der Geschwindigkeit (S) des Substratverbrauchs,
- Berechnung des Verhältnisses L/S und
- Steuerung der Substratkonzentration ($c_s$) im Kulturmedium über die Zufuhr von Substrat mit Hilfe des Steuerventils (7) derart, daß das Verhältnis L/S innerhalb oder unterhalb eienes gegebenen oberen Grenzwerts (A) gehalten wird.

15. Vorrichtung nach Anspruch 14 mit
- einer Einrichtung (6, 13) zur Lieferung von Sauerstoff zur Fermentiereinrichtung (1) mit einer Sauerstofflieferleitung (13) und einem Steuerventil (6), das in der Leitung (13) angeordnet ist und durch die Betriebssteuereinrichtung (12) gesteuert wird.

16. Vorrichtung nach Anspruch 14 oder 15, dadurch gekennzeichnet, daß die Erfassungseinrichtung (8) für die Substratkonzentration eine die Glucosekonzentration erfassende Einrichtung ist.

17. Vorrichtung nach einem der Ansprüche 14 bis 16, dadurch gekennzeichnet, daß die Betriebssteuereinrichtung (12) die Entleerung des Kulturmediums und die Abscheidung des Überstehenden des Kulturmediums steuern kann mit Hilfe des Zellen/Kulturmedium-Abscheiders (10) auf der Basis der Ausgangssignale der Erfassungseinrichtung (9) für die Milchsäurekonzentration und der Erfassungseinrichtung (8) für die Substratkonzentration.

**Revendications**

1. Procédé pour commander les conditions de culture de cellules animales dans un milieu de culture contenant un substrat, caractérisé par :
- la mesure de la concentration en acide lactique ($c_\ell$) et de la concentration en substrat ($c_s$) du milieu de culture à des instants prédéterminés définissant des durées prédéterminées ($\Delta t$), ou en continu,
- le calcul de la vitesse (L) de formation de l'acide lactique à partir de la variation de concentration ($\Delta c_\ell$) au cours des durées ($\Delta t$) entre des instants prédéterminés subséquents sous la forme $\Delta c_\ell/\Delta t$, ou en continu sous la forme $dc_\ell/dt$,
- le calcul de la vitesse (S) de consommation du substrat à partir de la variation de concentration ($\Delta c_s$) au cours des mêmes durées prédéterminées ($\Delta t$) sous la forme $\Delta c_s/\Delta t$, ou en continu sous la forme $dc_s/dt$,
- le calcul du rapport L/S respectif selon les relations $L/S \approx \Delta c_\ell/\Delta c_s$ ou $L/S = dc_\ell/dc_s$, et
- la commande de la concentration en oxygène dissous et/ou de la concentration en substrat ($c_s$) dans le milieu de culture de manière à maintenir le rapport L/S à ou sous une valeur limite supérieure (A) prédéterminée qui dépend du type des cellules animales cultivées.

2. Procédé selon la revendication 1, caractérisé en ce que le glucose est utilisé comme substrat.

3. Procédé selon la revendication 1 ou 2, caractérisé en ce que la concentration en oxygène dissous et/ou la concentration en substrat ($c_s$) sont commandées par apport par étapes d'oxygène et/ou de substrat au milieu de culture à des instants prédéterminés, et le rapport L/S est déterminé pour chaque durée ($\Delta t$) entre ces instants prédéterminés.

4. Procédé selon l'une quelconque des revendications 1 à 3, caractérisé en ce que la commande de la concentration en oxygène dissous et/ou de la concentration en substrat ($c_s$) dans le milieu de culture est accomplie lorsque le rapport L/S est ou tend à être supérieur à la valeur limite (A) prédéterminée.

5. Procédé selon l'une quelconque des revendications 1 à 4, caractérisé en ce que la valeur limite supérieure (A) est égale à 0,6.

6. Procédé selon l'une quelconque des revendications 2 à 5, caractérisé en ce que la commande est accomplie par augmentation de la concentration en oxygène dissous et/ou par diminution de la concentration en glucose ($c_g$) dans le milieu de culture.

7. Procédé selon l'une quelconque des revendications 1 à 6, caractérisé en ce que de l'oxygène est fourni au milieu de culture en continu pendant toute la durée de la culture.

8. Procédé selon l'une quelconque des revendications 1 à 7, caractérisé en ce que, pendant toute la durée de la culture, les cellules animales sont périodiquement séparées du milieu de culture et placées dans un nouveau milieu de culture, de préférence dans un nouveau milieu de culture ayant une concentration en substrat/glucose supérieure à celle du milieu de culture utilisé.

9. Procédé selon l'une quelconque des revendications 1 à 8, caractérisé en ce que la commande est accomplie par réduction de la concentration en substrat/glucose pour réduire la croissance des cellules et induire l'assimilation de l'acide lactique lorsque la concentration en acide lactique ($c_l$) du milieu de culture atteint une valeur prédéterminée, de préférence 2,5 g/l.

10. Procédé selon la revendication 9, caractérisé en ce que, pendant toute la culture, du substrat/glucose est ajouté jusqu'à ce que la concentration en acide lactique ($c_l$) atteigne la valeur prédéterminée.

11. Procédé selon l'une quelconque des revendications 1 à 10, caractérisé en ce que la commande est accomplie par réduction de la concentration en substrat/glucose par commande ou par arrêt de l'addition du substrat/glucose.

12. Procédé selon l'une quelconque des revendications 1 à 11, caractérisé par l'utilisation de cellules animales d'un type exprimant un gène d'une enzyme qui assimile l'acide lactique, de préférence d'une lactate déshydrogénase de type cardiaque.

13. Procédé selon l'une quelconque des revendications 1 à 12, caractérisé en ce que l'on utilise des cellules animales provenant du foie.

14. Appareil pour commander les conditions de culture de cellules animales dans un milieu de culture contenant un substrat, en particulier pour mettre en oeuvre le procédé selon l'une quelconque des revendications 1 à 13, comprenant :
   - un fermenteur (1) pour le milieu de culture (2), muni de préférence d'un agitateur (4),
   - un réservoir à substrat (5) relié au fermenteur (1) au moyen d'une conduite (14) comprenant une vanne de commande (7),
   - un séparateur cellules/milieu de culture (10) disposé du côté évacuation du fermenteur (1),
   - une pompe à surnageant de milieu de culture (11) disposée dans la conduite d'évacuation (17) du fermenteur (1),
   - un dispositif de détection de la concentration en acide lactique (9) disposé près de ou dans le fermenteur (1),
   - un dispositif de détection de la concentration en substrat (8) disposé près de ou dans le fermenteur (1), et
   - un dispositif de commande de fonctionnement (12) pour commander la vanne de commande (7) et recevoir les signaux de sortie du dispositif de détection de la concentration en acide lactique (9) et du dispositif de détection de la concentration en substrat (8), et conçu
   - pour déterminer la vitesse (L) de formation de l'acide lactique,
   - pour déterminer la vitesse (S) de consommation du substrat,
   - pour calculer le rapport L/S, et
   - pour commander la concentration en substrat ($c_s$) dans le milieu de culture par le biais de l'apport de substrat au moyen de la vanne de commande (7) de manière à maintenir le rapport L/S à ou sous une valeur limite supérieure (A) prédéterminée.

15. Appareil selon la revendication 14, comprenant en outre :
   - un dispositif (6, 13) pour fournir de l'oxygène au fermenteur (1), comprenant une conduite de fourniture d'oxygène (13) et une vanne de commande (6) disposée dans la conduite (13) et commandée par le dispositif de commande de fonctionnement (12).

16. Appareil selon la revendication 14 ou 15, caractérisé en ce que le dispositif de détection de la concentration en substrat (8) est un dispositif de détection de la concentration en glucose.

17. Appareil selon l'une quelconque des revendications 14 à 16, caractérisé en ce que le dispositif de commande de fonctionnement (12) est conçu pour commander l'évacuation du milieu de culture et la séparation du surnageant du milieu de culture au moyen du séparateur cellules/milieu de culture (10) sur la base des signaux de sortie du dispositif de détection de la concentration en acide lactique (9) et du dispositif de détection de la concentration en substrat (8).

EP 0 387 840 B1

Fig. 1(a)

Fig. 1(b)

15

Fig 2(a)

Fig 2(b)

Fig 3(a)

Fig 3.(b)

Fig 4(a)

Fig 4(b)

Fig 5(a)

Fig 5(b)

Fig. 6

Fig 7(a)

Fig 7(b)

Lactic acid
concentration
(mg/l)

Fig. 8(a)

Gluc ose
concentration
(mg/l)

Fig. 8(b)

Cell
concentration
(cells/ml)

Fig 8(c)

Culture time (d)

Fig. 9

Fig. 10

Lactic acid concentration (mg/l)

Glucose concentration (mg/l)

Cell concentration (cells/ml)

Culture time (d)